# EUROPÄISCHE PATENTSCHRIFT

(11) **EP 1 062 969 B1**
(45) Veröffentlichungstag und Bekanntmachung des Hinweises auf die Patenterteilung: **16.04.2008**
(21) Anmeldenummer: 00250196.3
(22) Anmeldetag: 23.06.2000
(51) Int. Cl.: A61B 5/0428, A61N 1/36, A61N 1/05

(54) **Elektrodenanordnung**
Electrode assembly
Ensemble électrode

(30) Priorität: 25.06.1999 DE 19930265
(43) Veröffentlichungstag der Anmeldung: 27.12.2000
(73) Patentinhaber: BIOTRONIK GmbH & Co. KG, 12359 Berlin (DE)
(72) Erfinder: Witte, Joachim, 10409 Berlin (DE)
(74) Vertreter: Lindner-Vogt, Karin L.

(56) Entgegenhaltungen:
- FR-A- 2 566 201
- US-A- 4 628 934
- US-A- 5 018 523
- US-A- 5 469 000

## Beschreibung

Die Erfindung betrifft eine implantierbare Elektrodenanordnung, die eine Elektrodenleitung mit mehreren elektrisch leitenden Oberflächenbereichen im Bereich des distalen Endes zur Abgabe elektrischer Signale an ein Herz und/oder zur Aufnahme von Signalen von einem Herzen umfaßt, die über mindestens eine elektrische Leitung der Elektrodenleitung mit einer elektrische Signale aufnehmenden und/oder Impulse abgebenden kardioelektrischen Vorrichtung für einen Defibrillator oder Herzschrittmacher verbindbar sind. Eine Elektrodenanordnung nach dem Oberbegriff des Anspruchs 1 ist aus US 4 628 934 bekannt.

Elektrodenanordnungen mit einer Elektrodenleitung und mehreren elektrisch leitenden Oberflächenbereichen beispielsweise von Tip- oder Ringelelektroden am distalen Ende der Elektrodenleitung sind beispielsweise aus der EP 0 571 797, der US 4,484,352 und der US 4,628,934 bekannt. Bei den bekannten Elektrodenanordnungen sind die als Stimulations- oder Abfühlelektroden dienenden elektrisch leitenden Oberflächenabschnitte einzeln mittels elektrischer Leitungen, die in der Elektrodenleitung verlaufen, mit einem Herzschrittmacher oder Defibrillator verbunden. In den genannten Druckschriften ist auch jeweils beschrieben, daß von der Vielzahl der Elektroden oder Elektrodenkombinationen die jeweils bestgeeigneten ausgewählt werden, um diese beispielsweise zur Stimulation eines menschlichen Herzens zu verwenden. Nachteilig an den bekannten Elektrodenanordnungen ist, daß sie regelmäßig nur zusammen mit speziell angepaßten Herzschrittmachern oder Defibrillatoren verwendet werden können, die es erlauben, am proximalen Ende der Elektrodenleitung sämtliche Zuleitungen zu kontaktieren, die zu den elektrisch leitenden Oberflächenbereichen führen.

Üblich sind Elektrodenanordnungen, bei denen in der Elektrodenleitung nur ein oder zwei elektrische Leiter verlaufen, je nachdem, ob die Elektrodenanordnung zur unipolaren oder zur bipolaren Stimulation vorgesehen ist.

Elektrodenleitungen mit einer Eindrahtverbindung zwischen dem proximalen Ende der Elektrodenleitung und den elektrisch leitenden Oberflächenbereichen am distalen Ende der Elektrodenleitung sind für die unipolare Stimulation geeignet, bei der Stimulationsimpulse zwischen den elektrisch leitenden Oberflächenbereichen am distalen Ende der Elektrodenleitung und eine Neutralelektrode wie beispielsweise einem Gehäuse des Herzschrittmachers abgegeben werden. Daneben ist auch die bipolare Stimulation bekannt, bei der die Stimulationsenergie zwischen verschiedenen der elektrisch leitenden Oberflächenbereiche am distalen Ende der Elektrodenleitung abgegeben wird. Für die bipolare Stimulation ist in der Elektrodenleitung eine Zweidrahtverbindung zwischen proximalem und distalem Ende vorgesehen, die über zwei separate elektrische Leitungen hergestellt wird.

Ziel der Erfindung ist es, die Vorteile von Elektrodenanordnungen mit einer Mehrzahl von elektrisch leitenden und einzeln ansteuerbaren Oberflächenbereichen auch für Herzschrittmacher oder Defibrillatoren mit herkömmlichen Ein- oder Zweidrahtanschlüssen verfügbar zu machen.

Dieses Ziel wird erfindungsgemäß mit einer Elektrodenanordnung laut dem unabhängigen Anspruch 1 erreicht.

Eine derartige Elektrodenanordnung kann herkömmlich als Ein- oder Zweidrahtleitung mit den üblichen elektrischen Anschlüssen für einen Herzschrittmacher oder Defibrillator ausgeführt sein. Die Steuersignale für die Steuereinrichtung zum Schalten der Verbindung zwischen einzelnen elektrisch leitenden Oberflächenbereichen und dem Herzschrittmacher oder Defibrillator werden vom Defibrillator beispielsweise als digitale Signale auf die zumindest eine elektrische Leitung in der Elektrodenleitung gegeben und so zu den Steuermitteln geführt. Diese decodieren jedes empfangene Steuersignal und schalten entsprechend Verbindungen zwischen der elektrischen Leitung und den elektrisch leitenden Oberflächenbereichen einzeln oder in Gruppen ein oder aus.

Eine derartige Elektrodenanordnung braucht im Minimalfall nur einen einzigen elektrischen Leiter, der vom proximalen Ende der Elektrodenleitung zu den Schaltmitteln und den elektrisch leitenden Oberflächenbereichen am distalen Ende der Elektrodenleitung führt. Das Bezugspotential für die Steuersignale kann dann beispielsweise über eine Neutralelektrode mit dem Gehäuse des Schrittmachers hergestellt und über einen der elektrisch leitenden Oberflächenbereiche für die Steuermittel zur Verfügung stehen.

Vorzugsweise sind die Schaltmittel so gestaltet und angeordnet, daß mittels der Schaltmittel die Verbindung zwischen mehreren und allen elektrisch leitenden Oberflächenbereichen und elektrischen Leitungen einzeln oder in Gruppen hergestellt oder getrennt werden kann. Dies kann beispielsweise dadurch geschehen, daß für jeden elektrisch leitenden Oberflächenbereich ein Schaltelement vorgesehen wird. Eine derartige Elektrodenanordnung erlaubt es, alle möglichen Elektrodenkombinationen zu schalten.

Vorzugsweise sind die Steuermittel derart durch Steuersignale über die elektrische Leitung ansteuerbar, daß die Verbindung zwischen mehreren oder allen elektrisch leitenden Oberflächenbereichen und der elektrischen Leitung einzeln oder in Gruppen hergestellt oder getrennt werden kann. Dazu umfassen die Steuermittel einen Decoder, der beispielsweise Digitalsignale zum Ein- oder Ausschalten der Elektrodenverbindungen decodieren und entsprechende Ein- oder Ausschaltsignale für die Schaltmittel umsetzen kann. Für eine Elektrodenleitung mit acht Elektroden können beispielsweise 8-Bit-Steuersignale verwendet werden, bei denen ein Bit immer genau einer Elektrode zugeordnet ist und der Wert des Bits - 1 oder 0 - dem geforderten Schaltzustand des entsprechenden Schaltmittels - an oder aus - entspricht. Die digitalen Steuersignale werden dabei so gewählt, daß sie eindeutig beispielsweise aufgrund ihrer Flankensteilheit, als Steuersignale identifizierbar sind, auch wenn sie von Herzsignalen oder von Spannungsimpulsen zur Abgabe an das Herz überlagert werden. Dazu weisen die Steuermittel vorzugsweise entsprechende Filter auf, die so gestaltet sind, daß sie nur Steuersignale an einen Decoder der Steuermittel weiterleiten und weitere, die Steuersignale überlagernde Signale zurückhalten.

Um Beschädigungen der Steuermittel für die Stimulationsimpulse zu verhindern, sind vorzugsweise Abschaltmittel vorgesehen, die auf an der elektrischen anliegende Stimulationsimpulse ansprechen und während des Anlegens solcher Impulse die Verbindung zwischen den Steuermitteln und der elektrischen Leitung unterbrechen.

Weiterhin ist vorzugsweise ein Energiespeicher vorgesehen, der zur Energieversorgung der Schalt- und/oder der Steuermittel mit diesen verbunden und über die elektrische Leitung aufladbar ist. Der Energiespeicher ist dabei vorzugsweise ein Kondensator. Der Kondensator kann ständig mit geringer Leistung über eine elektrische Eindraht- oder Zweidrahtleitung der Elektrodenleitung geladen werden und kann den Steuer- bzw. Schaltmitteln bei Bedarf auch größere Energien zur Verfügung stellen, als über die elektrische Leitung verfügbar ist. Im Falle einer Eindrahtleitung wird der Kondensator über diese Eindrahtleitung und einen der elektrisch leitenden Oberflächenbereiche und ein als Neutralelektrode dienendes Gehäuse eines Herzschrittmachers geladen.

Die Schaltmittel sind vorzugsweise Leistungs-Feldeffekttransistoren. Zum Schalten derartiger Transistoren ist praktisch keine Leistung erforderlich. Darüberhinaus haben solche Transistoren im durchgeschalteten Zustand nur einen geringen ohmschen Widerstand.

Die Steuermittel umfassen vorzugsweise elektrisch lösch- oder programmierbare Lesespeicher, sogenannte EEPROMs. Auf diese Weise können die Steuermittel leicht programmiert werden, d.h. es kann leicht bestimmt werden, welche Art von Steuersignalen welchen Schaltzustand die Verbindung zwischen den einzelnen elektrisch leitenden Oberflächenbereichen und der Elektrodenleitung bewirken.

Ein Herzschrittmacher oder Defibrillator benötigt zum Betreiben einer derartigen Elektrodenanordnung außer einem Signalgeber für die Steuersignale für die Steuermittel der Elektrodenanordnung keine weiteren Bestandteile. Ein derartiger Signalgeber enthält vorzugsweise einen Speicher für bevorzugte Elektrodenkombinationen und die entsprechenden Steuersignale.

Die Erfindung soll nun anhand von Ausführungsbeispielen mit Hilfe der Figuren näher erläutert werden. Diese zeigen:
- Figur 1: Das distale Ende einer Elektrodenleitung mit elektrisch leitenden Oberflächenbereichen als Elektroden;
- Figur 2: Einen Längsschnitt durch einen Teil der in Figur 1 abgebildeten Elektrodenleitung.

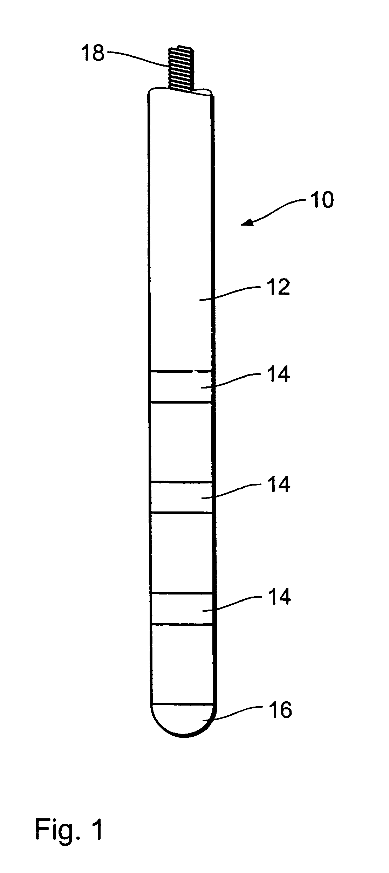

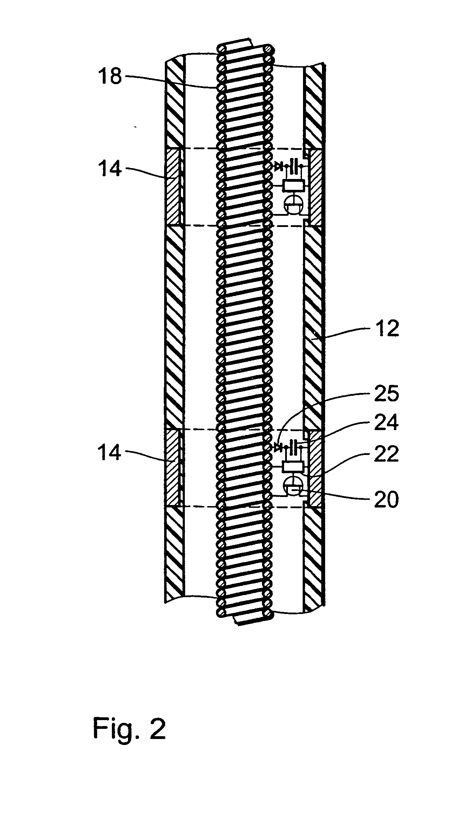

Das in Figur 1 dargestellte distale Ende umfaßt eine Elektrodenleitung 10 mit einer isolierenden Hülle 12 und darin eingelassenen Ringelektroden 14 sowie einer Tipelektrode 16 am äußersten Ende der Elektrodenleitung 10. Im Inneren der Elektrodenleitung 10 verläuft eine gewendelte elektrische Leitung 18, die zum Übertragen elektrischer Signale von und zu den Elektroden 14 und 16 dient. Die Elektroden 14 und 16 können beispielsweise aus Metall bestehen, wesentlich ist, daß die Elektroden 14 und 16 elektrisch leitende Oberflächenbereiche der Elektrodenleitung 10 bilden, die zumindest teilweise mit der elektrischen Leitung 18 verbindbar sind. Der Aufbau der Elektrodenleitung 10 ist dem in Figur 2 abgebildeten Längsschnitt durch die Elektrodenleitung 10 im Bereich zweier Ringelektroden 14 zu entnehmen. Zu erkennen ist die gewendelte elektrische Leitung 18, die innerhalb der isolierenden Hülle 12 verläuft. Zwischen der Leitung 18 und jeder Ringelektrode 14 sind folgende elektrische Bauelemente angeordnet: jeweils ein Feldeffekttransistor 20, ein Decoder bzw. eine Steuereinheit 22, sowie ein Kondensator 24 mit dazu in Serie geschalteter Gleichrichterdiode 25. Die Feldeffekttransistoren 20 sind Leistungstransistoren mit geringerem Durchgangswiderstand im durchgeschalteten Zustand. Sie stellen jeweils ein Schaltmittel zum Verbinden oder Unterbrechen einer elektrischen Verbindung zwischen der Leitung 18 und einer jeweiligen Ringelektrode 14 dar. Angesteuert werden die Feldeffekttransistoren 20 von der Steuereinheit bzw. dem Decoder 22. Die Steuereinheit bzw. der Decoder 22 haben zwei Steuereingänge, von denen einer mit der elektrischen Leitung 18 und der andere mit jeweils einer Ringelektrode 14 verbunden ist. Über die Steuereingänge kann die Steuereinheit 22 Steuersignale aufnehmen, die beispielsweise von einem Herzschrittmacher über die elektrische Leitung 18 sowie einer Neutralelektrode wie das Herzschrittmachergehäuse abgegeben werden und sich dementsprechend als Potentialfolge darstellen, die zwischen der Leitung 18 und einer Ringelektrode 14 liegt. Die Ringelektrode 14 ist dabei über einem Körper, in den sie eingesetzt ist, mit der Neutralelektrode verbunden.

Der Energieversorgung der Steuereinheit 22 dient ein Kondensator 24, der ebenfalls zwischen der Leitung 18 und jeweils einer Ringelektrode 14 angeordnet ist. Dieser Kondensator 24 wird über die elektrische Leitung und die Diode 25 einerseits sowie andererseits über die Ringelektrode 14 und die mit dieser korrespondierenden Neutralelektrode (nicht dargestellt) vom Herzschrittmacher mit Energie versorgt und hochohmig geladen.

Über entsprechende Steuersignale können beliebige der Elektroden 14 und 16 mittels der Steuereinheiten 22 und der Feldeffekttransistoren 20 mit der Leitung 18 verbunden oder von ihr getrennt werden.

Die in Figur 2 dargestellten Steuermittel enthalten EEPROMs, also elektrisch lösch- und programmierbare Lesespeicher, die über entsprechende Steuersignale konfiguriert werden und für die gewünschte Zuordnung von Steuersignalen und Verbindungen zwischen den Elektroden 14 und 16 und der Leitung 18 sorgen.

In die Steuereinheit 22 sind außerdem Abschaltmittel integriert, die auf Stimulationsimpulse ansprechen, die an der elektrischen Leitung 18 anliegen und die Verbindung zu beispielsweise einem in der Steuereinheit 22 integrierten Decoder während der Dauer solcher Stimulationsimpulse unterbrechen.

## Patentansprüche

1. Implantierbare Elektrodenanordnung umfassend eine Elektrodenleitung (10) mit mehreren elektrisch leitenden Oberflächenbereichen im Bereich des distalen Endes der Elektrodenleitung (10) zur Abgabe elektrischer Signale an ein Herz und/oder zur Aufnahme von Signalen von einem Herzen, die über mindestens eine elektrische Leitung (18) der Elektrodenleitung (10) mit einer elektrische Signale aufnehmenden und/oder Impulse abgebenden kardioelektrischen Vorrichtung wie einem Defibrillator oder einem Herzschrittmacher verbindbar sind,
in der Elektrodenleitung (10) angeordnete Schaltmittel, die mit der mindestens einen elektrischen Leitung (18) und mit mindestens einem elektrisch leitenden Oberflächenbereich verbunden und derart gestaltet sind, daß sie eine Verbindung zwischen dem elektrisch leitenden Oberflächenbereich und der kardioelektrischen Vorrichtung im Bereich der Elektrodenleitung (10) herstellen oder trennen können, **gekennzeichnet**
**durch** in der Elektrodenleitung (10) angeordnete Steuermittel, die mit der elektrischen Leitung (18) zur Aufnahme von Steuersignalen sowie mit den Schaltmitteln zum Umschalten von einem die Verbindung zwischen der elektrischen Leitung (18) und dem elektrisch leitenden Oberflächenbereich trennenden Zustand in einen die Verbindung herstellenden Zustand der Schaltmittel oder umgekehrt verbunden sind.

2. Elektrodenanordnung nach Anspruch 1, **dadurch gekennzeichnet, daß** mittels der Schaltmittel die Verbindung zwischen mehreren oder allen elektrisch leitenden Oberflächenbereichen und der elektrischen Leitung (18) einzeln oder in Gruppen Herstell- oder trennbar ist.

3. Elektrodenanordnung nach Anspruch 2, **dadurch gekennzeichnet, daß** die Steuermittel derart durch Steuersignale über die elektrische Leitung (18) ansteuerbar sind, daß die Verbindung zwischen mehreren oder allen elektrisch leitenden Oberflächenbereichen und der elektrischen Leitung (18) einzeln oder in Gruppen Herstell- oder trennbar ist.

4. Elektrodenanordnung nach einem der Ansprüche 1 bis 3, **gekennzeichnet durch** mindestens einen Energiespeicher, der zur Energieversorgung mit den Schaltmitteln und/oder den Steuermitteln verbunden und über die elektrische Leitung (18) aufladbar ist.

5. Elektrodenanordnung nach Anspruch 4, **dadurch gekennzeichnet, daß** der Energiespeicher ein elektrischer Kondensator ist.

6. Elektrodenanordnung nach einem der Ansprüche 1 bis 5, **dadurch gekennzeichnet, daß** die Schaltmittel Leistungs-Feldeffekttransistoren umfassen.

7. Elektrodenanordnung nach einem der Ansprüche 1 bis 6, **dadurch gekennzeichnet, daß** die Steuermittel elektrisch lösch- und programmierbare Lesespeicher (EEPROMs) enthalten.

## Claims

1. An implantable electrode configuration comprising an electrode line (10) having multiple electrically conductive surface areas in the area of the distal end of the electrode line (10) for delivering electrical signals to a heart and/or for recording signals from a heart, which are connectable via at least one electrical line (18) of the electrode line (10) to a cardioelectric device, such as a defibrillator or a cardiac pacemaker, which records electrical signals and/or delivers pulses, and
switching means, situated in the electrode line (10), which are connected to the at least one electrical line (18) and to at least one electrically conductive surface area and are designed in such a way that they may establish or disconnect a connection between the electrically conductive surface area and the cardioelectric device in the area of the electrode line (10),
**characterized by** control means situated in the electrode line (10), which are connected to the electrical line (18) for recording control signals and to the switching means for switching over from a state disconnecting the connection between the electrical line (18) and the electrically conductive surface area into a state of the switching means establishing the connection or vice versa.

2. The electrode configuration according to Claim 1, **characterized in that**, using the switching means, the connection between multiple or all electrically conductive surface areas and the electrical line (18) may be established or disconnected individually or in groups.

3. The electrode configuration according to Claim 2, **characterized in that** the control means are activatable by control signals via the electrical line (18) in such a way that the connection between multiple or all electrically conductive surface areas and the electrical line (18) may be established or disconnected individually or in groups.

4. The electrode configuration according to one of Claims 1 through 3, **characterized by** at least one power accumulator, which is connected to the switching means and/or the control means for power supply and is chargeable via the electrical line (18).

5. The electrode configuration according to Claim 4, **characterized in that** the power accumulator is an electrical capacitor.

6. The electrode configuration according to one of Claims 1 through 5, **characterized in that** the switching means comprise power field-effect transistors.

7. The electrode configuration according to one of Claims 1 through 6, **characterized in that** the control means contain electrically erasable and programmable read-only memories (EEPROMs).

## Revendications

1. Agencement d'électrodes implantable comprenant une ligne d'électrodes (10) avec plusieurs zones de surfaces électroconductrices dans la zone de l'extrémité distale de la ligne d'électrodes (10) pour la délivrance de signaux électriques à un coeur et/ou pour la réception de signaux provenant d'un coeur, qui peuvent être reliées au moyen d'au moins une ligne (18) électrique de la ligne d'électrodes (10) à un dispositif cardioélectrique recevant des signaux électriques et/ou délivrant des impulsions tel qu'un défibrillateur ou un pacemaker,
des moyens de commutation disposés dans la ligne d'électrodes (10), qui sont reliés à la au moins une ligne (18) électrique et à au moins une zone de surface électroconductrice et sont conçus de telle sorte qu'ils peuvent établir ou interrompre une liaison entre la zone de surface électroconductrice et le dispositif cardioélectrique dans la zone de la ligne d'électrodes (10), **caractérisé**
**par** des moyens de commande disposés dans la ligne d'électrodes (10), qui sont reliés à la ligne (18) électrique pour la réception de signaux de commande ainsi qu'aux moyens de commutation pour la commutation d'un état séparant la liaison entre la ligne (18) électrique et la zone de surface électroconductrice dans un état des moyens de commutation qui établit la liaison ou inversement.

2. Agencement d'électrodes selon la revendication 1, **caractérisé en ce que** la liaison entre plusieurs ou l'ensemble des zones de surfaces électroconductrices et la ligne (18) électrique peut être établie ou interrompue au moyen des moyens de commutation de façon individuelle ou en groupe.

3. Agencement d'électrodes selon la revendication 2, **caractérisé en ce que** les moyens de commande peuvent être activés par des signaux de commande par la ligne (18) électrique de telle sorte que la liaison entre plusieurs ou l'ensemble des zones de surfaces électroconductrices et la ligne (18) électrique peut être établie ou séparée de façon individuelle ou en groupe.

4. Agencement d'électrodes selon l'une quelconque des revendications 1 à 3, **caractérisé par** au moins un accumulateur d'énergie, qui est relié pour l'alimentation en énergie aux moyens de commutation et/ou aux moyens de commande et peut être chargé par la ligne (18) électrique.

5. Agencement d'électrodes selon la revendication 4, **caractérisé en ce que** l'accumulateur d'énergie est un condensateur électrique.

6. Agencement d'électrodes selon l'une quelconque des revendications 1 à 5, **caractérisé en ce que** les moyens de commutation comprennent des transistors à effet de champ de puissance.

7. Agencement d'électrodes selon l'une quelconque des revendications 1 à 6, **caractérisé en ce que** les moyens de commande contiennent des mémoires de lecture pouvant être effacées et programmées électriquement (EEPROMs).
